# EUROPÄISCHE PATENTSCHRIFT

(11) **EP 1 787 649 B1**
(45) Veröffentlichungstag und Bekanntmachung des Hinweises auf die Patenterteilung: **11.03.2009**
(21) Anmeldenummer: 05022324.7
(22) Anmeldetag: 13.10.2005
(51) Int. Cl.: A61K 31/565, A61K 31/57, A61P 15/00, A61P 15/18

(54) **Verwendung von Estradiolvalerat in Kombination mit Dienogest zur oralen Therapie der dysfunktionellen uterinen Blutung in Einheit mit einer oralen Kontrazeption**
Use of estradiolvalerate and dienogest for oral treatment of dysfunctional uterine bleeding in a contraceptive method
Utilisation d'une combinaison de valérate d'oestradiol et dienogest en tant que contraceptif oral, pour le traitement de l'hémorragie utérine dysfonctionnelle

(43) Veröffentlichungstag der Anmeldung: 23.05.2007
(73) Patentinhaber: Bayer Schering Pharma Aktiengesellschaft, 13353 Berlin (DE)
(72) Erfinder: Zeun, Susan, 10245 Berlin (DE); Boudes, Pol, Hackettstown, NJ 07840 (US); Endrikat, Jan, H9H 5B5 Québec (CA); Secci, Angelo, Parsippany, NJ 07054 (US); Zimmermann, Holger, 14612 Falkensee (DE)

(56) Entgegenhaltungen:
- EP-A- 0 696 454
- EP-A- 0 770 388
- WO-A-20/05102247
- GRASER T ET AL: "Continuous-combined treatment of the menopause with combinations of oestradiol valerate and dienogest: A dose-ranging study" MATURITAS, Bd. 35, Nr. 3, 30. Juni 2000 (2000-06-30), Seiten 253-261, XP002369505 ISSN: 0378-5122
- VON SCHOULTZ B: "Clinical efficacy and safety of combined estradiol valerate and dienogest: a new no-bleed treatment." CLIMACTERIC : THE JOURNAL OF THE INTERNATIONAL MENOPAUSE SOCIETY. AUG 2003, Bd. 6 Suppl 2, August 2003 (2003-08), Seiten 24-32, XP009062446 ISSN: 1369-7137
- DAVIS A ET AL: "Triphasic Norgestimate-Ethinyl Estradiol for Treating Dysfunctional Uterine Bleeding" OBSTETRICS AND GYNECOLOGY, NEW YORK, NY, US, Bd. 96, Nr. 6, Dezember 2000 (2000-12), Seiten 913-920, XP002317447 ISSN: 0029-7844

## Beschreibung

### Gebiet der Erfindung

Die Erfindung betrifft die Verwendung von Estradiolvalerat in Kombination mit 17α-Cyanomethyl-17-β-hydroxyestra-4,9-dien-3on (Dienogest), dabei enthaltend eine erste Phase aus 2 Tagesdosiseinheiten des Estradiolvalerat zu 3 mg, eine zweite Phase aus 2 Gruppen von Tagesdosiseinheiten , wobei die erste Gruppe 5 Tagesdosiseinheiten einer Kombination von 2 mg Estradiolvalerat und 2 mg Dienogest und die zweite Gruppe 17 Tagesdosiseinheiten einer Kombination von 2 mg Estradiolvalerat und 3 mg Dienogest enthält,
eine dritte Phase aus 2 Tagesdosiseinheiten mit 1mg Estradiolvalerat und eine weitere Phase aus 2 Tagesdosiseinheiten an pharmazeutisch unbedenklichem Placebo zur Herstellung eines mehrphasigen Kombinationspräparates mit einer Gesamtzahl von 28 Tagesdosiseinheiten zur oralen Therapie der dysfunktionellen uterinen Blutung, worunter eine verlängerte Menstruationsblutung größer 7 Tage zu verstehen ist, in Einheit mit einer oralen Kontrazeption.
Die gesamten Tagesdosiseinheiten der mehrphasigen Kombination und des pharmazeutisch unbedenklichem Placebo entsprechen 28 Tagen.

### Allgemeiner Stand der Technik

Die dysfunktionelle uterine Blutung (DUB) ist ein häufiges klinisches Problem in der Gynäkologie und betrifft bis zu 33 % der ambulanten gynäkologischen Arztbesuche (Awwad JT, Toth TL, Schiff I. Abnormal uterine bleeding in the perimenopause. Int J Fertil 1993;38:261-9). DUB Symptome sind:
- verlängerte Mensturationsblutung (> 7 Tage)
- häufige Blutung (Intervall zwischen den Blutungen von kleiner oder gleich 21 Tage)
- verstärkte Blutung (größer oder gleich 80 ml)

DUB tritt sowohl in Verbindung mit einer Anovulation, als auch mit einer Ovulation auf. Grundlegend für derartige Blutungsstörung ist eine Imbalance zwischen der östrogen-stimulierten Aufbauphase (Proliferation) des Endometrium und der gestagenen Umwandlung des Endometriums. Resultieren die DUB Symptome aus einer chronischen Anovulation ist das Endometrium häufig einer gesteigerten östrogenen Proliferation ausgesetzt. Diese kann, neben den benannten Blutungsstörungen zur Hyperplasie des Endometriums führen. (Speroff et all, Clinical Gynecologic Endocrinology and Infertility, Sixth Edition. Lippincott Williams& Wilkins, 1999).
Die Hyperplasie des Endometriums ist ein Risikofaktor für die Entstehung von Endometriumskrebs.

Fraser, I. S., Aust.NZ J Obstet Gynaecol (1990) 30(4),353-356 offenbart die Behandlung der dysfunktionellen uterinen Blutung mit einer Gabe von 5 mg Norethisteron, 3mal täglich oder 10 mg Medroxyprogesteronacetateines 3mal täglich als alleiniges hoch dosiertes Gestagen jeweils für 14 Tage vom 12. bis 25. Zyklustag in 6 anovulatorischen Frauen und über 20 Tage vom 5. bis 25. Zyklustag in 10 ovulatorischen Frauen. In beiden Gruppen konnte eine Verringerung der Blutungsdauer erreicht werden. Eine zuverlässige Kontrazeption war nicht gegeben.

Hickey M, Higham J und Fraser IS The Chochrane Libary, Issue 3 2004 (Hickey M, Higham J, Fraser IS. Progestogens versus oestrogens and progestogens for irregular uterine bleeding associated with anovulation (Cochrane Review). In: The Cochrane Library, Issue 3, 2004. Chichester, UK: John Wiley & Sons, Ltd) beschreiben in einem Review Artikel die niedrige Toleranz der Frauen gegenüber irregulären und starken Blutungen. Sie beschreiben die Rationale der Anwendung von Gestagenen um eine Umwandlung des Endometriums zu erreichen und somit stabilere Menstruationszyklen zu schaffen. Schlussfolgerung des Artikel ist, das derzeit klinische Daten aus randomisierten Studien fehlen um die Wirksamkeit der beschriebenen Behandlungsmöglichkeiten nachzuweisen.

EP 0770388-A1 offenbart ein Mehrphasenpräparat, enthaltend u.a. Estradiolvalerat und Dienogest, welches gegenüber gattungsgemäßen herkömmlichen ovulationshemmenden Mitteln eine hohe kontrazeptive Sicherheit realisiert und das zyklische Blutungsverhalten verbessert. Auch EP 0696454-A2 offenbart ein mehrphasisches Kombinationspräparat zur Kontrazeption/Hormonsubstitution, welches dreistufig über 28 Tagesdosiseinheiten ausgelegt ist, wobei das Estradiolvalerat kontinuierlich und fortlaufend über die Gesamtzahl der Tagesdosiseinheiten angewendet wird.
Gräser, T. et al , "Continousa-combined treatment of menopause" erklärt in MATURITAS 35 (2000) 253-261, dass eine einphasische kontinuierliche Behandlung der dysfunktionellen uterinen Blutung mit durchgängig verabreichtem Dienogest/Estradiolvalerat besonders erfolgreich ist.
Dies bestätigt auch Von Schoultz, B., "Clinical efficacy..." in CLIMAC-TERIC, 2003, 6 (Suppl. 2), 24-32.

WO 2005/102247-A2 offenbart ebenfalls ein Mehrphasenpräparat zur Kontrazeption auf Basis eines natürlichen Estrogens mit einem synthetischen Gestagen, u.a. Dienogest, welches in erster Linie eine hohe kontrazeptive Sicherheit realisiert und auch das zyklische Blutungsverhalten verbessert.

Steiner, R, Schweiz Rundsch Med Prax. (2000) 91 (46), 1967-1974 zeigt ebenfalls auf, dass die Behandlung der dysfunktionellen uterinen Blutung u.a. mit hoch dosierten Gestagenen, Estrogenen oder einer Kombinationen von Beiden erfolgen sollte. Ein Anwendungsregime sieht Steiner in der oralen Gabe von 0,01 mg Ethinylestradiol mit 2 mg Norethisteronacetat für 8 Tage in absteigender Dosierung, nämlich 6,5,4,3,3,3,3,3/Tag. Neben der hormonellen Beeinflussung postuliert Steiner die Möglichkeit der Behandlung in einer akuten Blutungssituation mit Tranexamsäure bis 4x2 Tabletten pro Tag.

Davis A, Obstet gynecol. (2000) 96(6), 913-920 zeigt die Behandlung von dysfunktionellen uterinen Blutungen mittels einer dreistufigen Gabe von Ethinylestradiol (EE)/Norgestimate (NGM) gefolgt von einer hormonfreien Placebo-Gabe für drei 28-Tage Zyklen auf. Das Behandlungsregime gliedert sich dabei derart, dass die Dosis an EE über 21 Tage konstant bleibt (0,035 mg EE), die Dosis an NGM über 21 Tage ansteigt (7 Tagesdosiseinheiten zu 0,180 mg NMG und 7 Tagesdosiseinheiten zu 0, 215 mg NMG und 7 Tagesdosiseinheiten zu 0, 250 mg NMG), gefolgt von einer 7tägigen hormonfreien Placebo-Gabe. In der von Davis durchgeführten placebo-kontrollierten Studie wurden 45% Frauen mit verstärkter Menstruationsblutung (Metrorrhagie, Menometrorrhagie und Polymenorrhoe) und circa 55% Frauen mit verminderter Menstruationsblutung (Oligomenorrhoe) eingeschlossen. Die höchste Erfolgsquote im Vergleich zu Placebo konnte in Frauen mit einer verminderten Menstruationsblutung erreicht werden, hier wurden regelmäßige Abbruchblutungen induziert. Die Oligomenorrhoe ist nicht notwendigerweise Bestandteil der Symptomengruppe DUB und als behandlungswürdige Erkrankung anerkannt.

US 6,797,282 erklärt in der Beschreibung allgemein, dass Langzeitanwendungen (3 Monate) oraler Kontrazeptiva zur Behandlung von Menorrhagien - eine Form von dysfunktioneller uteriner Blutung angewendet werden können.

### Detaillierte Offenbarung der Erfindung

Der Erfindung liegt die Aufgabe zugrunde, Mittel zur Behandlung von dysfunktionellen uterinen Blutungen zu entwickeln, die generell die Menge der Blutung verringern, und dass Wiederauftreten einer dysfunktionellen Blutung verhinderrn und gleichzeitig eine zuverlässige, sichere und gut verträgliche orale Kontrazeption sichern. Unter dem Begriff " dysfunktionelle uterine Blutung" ist hier die verlängerte Menstruationsblutung, größer 7 Tage, jeweils ohne organische Ursache zu verstehen.

Die Aufgabe wird erfindungsgemäß durch die Verwendung von Estradiolvalerat in Kombination mit 17α-Cyanomethyl-17-β-hydroxyestra-4,9-dien-3on (Dienogest) zur Herstellung eines mehrphasigen Kombinationspräparates zur oralen Therapie der dysfunktionellen uterinen Blutung in Einheit mit einer oralen Kontrazeption gelöst. Die Kombination von Estradiolvalerat mit Dienogest umfasst dabei eine erste Phase aus 2 Tagesdosiseinheiten des Estradiolvalerat zu 3 mg, eine zweite Phase aus 2 Gruppen von Tagesdosiseinheiten , wobei die erste Gruppe 5 Tagesdosiseinheiten einer Kombination von 2 mg Estradiolvalerat und 2 mg Dienogest und die zweite Gruppe 17 Tagesdosiseinheiten einer Kombination von 2 mg Estradiolvalerat und 3 mg Dienogest enthält, eine dritte Phase aus 2 Tagesdosiseinheiten mit 1mg Estradiolvalerat und eine weitere Phase aus 2 Tagesdosiseinheiten an pharmazeutisch unbedenklichem Placebo. Die gesamten Tagesdosiseinheiten der mehrphasigen Kombination und des pharmazeutisch unbedenklichem Placebo entsprechen 28 Tagen.

Die Dauer der Anwendung umfasst mindestens einen Einnahmezyklus und ist vom individuellen Wunsch der Frau nach Kontrazeption abhängig.

### Untersuchungen zur Wirksamkeit der beanspruchten Formulierung

In einer randomisierten, doppelt-blinden, placebo-kontrollierten, klinischen Studie werden 180 Frauen mit DUB Symptomen und nach Ausschluss einer organischen Ursache der Symptome mit geeigneten diagnostischen Methoden (transvaginaler Ultraschall, Bestimmung von Hormonen im Blut) im Alter zwischen 18 und 50 Jahren, die ihr schriftliches Einverständnis zur Studienteilnahme gegeben haben, behandelt. 120 Frauen erhalten Estradiolvalerat und Dienogest entsprechend der beanspruchten Kombination und 60 Frauen ein Placebo.
Der Versuch umfasst eine Run-in Phase von 90 Tagen in denen die Schwere der Blutungsstörungen aufgenommen werden, 6 Behandlungszyklen und einen Nachbehandlungszyklus (Follow-up-Phase).

Die Blutungsstärke wird durch die alkaline Hämatinmethode quantitativ erfasst. Hierzu werden die Frauen über die gesamte Studie die benutzte Monatshygiene sammeln und beim Prüfzentrum abgeben. Die Dauer der Blutung und die Dauer der blutungsfreien Intervalle werden über tägliche Dokumentation in einem elektronischen Tagebuch erfasst.

## Patentansprüche

1. Verwendung von Estradiolvalerat in Kombination mit 17α-Cyanomethyl-17-β-hydroxyestra-4,9-dien-3on (Dienogest), dabei enthaltend
eine erste Phase aus 2 Tagesdosiseinheiten des Estradiolvalerat zu 3 mg,
eine zweite Phase aus 2 Gruppen von Tagesdosiseinheiten , wobei die erste Gruppe 5 Tagesdosiseinheiten einer Kombination von 2 mg Estradiolvalerat und 2 mg Dienogest
und die zweite Gruppe 17 Tagesdosiseinheiten einer Kombination von 2 mg Estradiolvalerat und 3 mg Dienogest enthält,
eine dritte Phase aus 2 Tagesdosiseinheiten mit 1 mg Estradiolvalerat und eine weitere Phase aus 2 Tagesdosiseinheiten an pharmazeutisch unbedenklichem Placebo
zur Herstellung eines mehrphasigen Kombinationspräparates mit einer Gesamtzahl von 28 Tagesdosiseinheiten zur oralen Therapie der dysfunktionellen uterinen Blutung, worunter eine Menstruationsblutung größer 7 Tage zu verstehen ist, in Einheit mit einer oralen Kontrazeption.

## Claims

1. Use of estradiol valerate in combination with 17α-cyanomethyl-17-β-hydroxyestra-4,9-dien-3one (dienogest), comprising
a first phase of 2 daily dose units of 3 mg of estradiol valerate,
a second phase of 2 groups of daily dose units, where the first group comprises 5 daily dose units of a combination of 2 mg of estradiol valerate and 2 mg of dienogest
and the second group comprises 17 daily dose units of a combination of 2 mg of estradiol valerate and 3 mg of dienogest,
a third phase of 2 daily dose units with 1 mg of estradiol valerate and a further phase of 2 daily dose units of pharmaceutically acceptable placebo
for producing a multiphase combination product having a total number of 28 daily dose units for oral therapy of dysfunctional uterine bleeding, meaning menstrual bleeding for more than 7 days, united with oral contraception.

## Revendications

1. Utilisation de valérate d'estradiol en combinaison avec de la 17α-cyanométhyl-17-β-hydroxyestra-4,9-dién-3-one (Dienogest), contenant
- une première phase de 2 unités de dosage journalier de valérate d'estradiol à raison de 3 mg,
- une deuxième phase de 2 groupes d'unités de dosage journalier, le premier groupe contenant 5 unités de dosage journalier d'une combinaison de 2 mg de valérate d'estradiol et 2 mg de Dienogest
- et le deuxième groupe contenant 17 unités de dosage journalier d'une combinaison de 2 mg de valérate d'estradiol et 3 mg de Dienogest
- une troisième phase de 2 unités de dosage journalier avec 1 mg de valérate d'estradiol et une autre phase de 2 unités de dosage journalier de placebo pharmaceutiquement inoffensif
pour la préparation d'une préparation combinée à plusieurs phases présentant un nombre total de 28 unités de dosage journalier destinée à la thérapie orale de l'hémorragie utérine dysfonctionnelle, ce qui signifie des règles pendant plus de 7 jours, ensemble avec une contraception orale.
